**Europäisches Patentamt**

(19) **European Patent Office**

**Office européen des brevets**

(11) Publication number : **0 485 504 B1**

(12) # EUROPEAN PATENT SPECIFICATION

(45) Date of publication of patent specification :
01.12.93 Bulletin 93/48

(51) Int. Cl.⁵ : **C12N 15/57, C12N 9/64, C12N 5/00**

(21) Application number : **90912701.1**

(22) Date of filing : **07.08.90**

(86) International application number :
**PCT/US90/04419**

(87) International publication number :
**WO 91/02065 21.02.91 Gazette 91/05**

(54) **CELL CULTURE METHODS FOR PRODUCING ACTIVATED PROTEIN C.**

(30) Priority : **11.08.89 US 392861**

(43) Date of publication of application :
**20.05.92 Bulletin 92/21**

(45) Publication of the grant of the patent :
**01.12.93 Bulletin 93/48**

(84) Designated Contracting States :
**AT BE CH DE DK ES FR GB IT LI LU NL SE**

(56) References cited :
**EP-A- 0 215 548**
**EP-A- 0 254 076**
**EP-A- 0 266 190**
**EP-A- 0 306 968**
**EP-A- 0 319 312**
**WO-A-87/01389**

(73) Proprietor : **ZYMOGENETICS, INC.**
**4225 Roosevelt Way N.E.**
**Seattle, WA 98108 (US)**

(72) Inventor : **KUMAR, Anur, Ashok**
**2352 Terraza Ribera**
**Carlsbad, CA 92009 (US)**
Inventor : **FOSTER, Donald, C.**
**3002 N.E. 181st Street**
**Seattle, WA 98155 (US)**

(74) Representative : **Brown, John David et al**
**FORRESTER & BOEHMERT**
**Franz-Joseph-Strasse 38**
**D-80801 München (DE)**

EP 0 485 504 B1

## Description

<u>Technical Field</u>

The present invention relates generally to plasma proteins and methods for producing those proteins, and more specifically to cell culture methods for producing proteins having substantially the same biological activity as human activated protein C.

<u>Background of the Invention</u>

Protein C is a zymogen, or precursor, of a serine protease that plays an important role in the regulation of blood coagulation and in the generation of fibrinolytic activity <u>in vivo</u>. It is synthesized in the liver as a single-chain polypeptide that undergoes considerable processing to give rise to a two-chain molecule comprising heavy ($M_r = 40,000$) and light ($M_r = 21,000$) chains held together by a disulfide bond. The circulating two-chain intermediate is converted to the biologically active form of the molecule, known as "activated protein C" (APC), by the thrombin-mediated cleavage of a 12-residue peptide (also know as the activation peptide) from the amino-terminus of the heavy chain. The cleavage reaction is augmented <u>in vivo</u> by thrombomodulin, an endothelial cell co-factor (Esmon and Owen, <u>Proc. Natl. Acad. Sci. USA</u> <u>78</u>:2249-2252, 1981).

Protein C is a glycoprotein that contains approximately nine residues of gamma-carboxyglutamic acid (Gla) and one equivalent of beta-hydroxyaspartic acid, which are formed by post-translational modifications of glutamic acid and aspartic acid residues, respectively. The post-translational formation of specific gamma-carboxyglutamic acid residues in protein C requires vitamin K. These unusual amino acid residues bind to calcium ions and are believed to be responsible for the interaction of protein C with phospholipid, which is required for the biological activity of protein C.

In contrast to the coagulation-promoting action of other vitamin K-dependent plasma proteins, such as factor VII, factor IX, and factor X, activated protein C acts as a regulator of the coagulation process through the inactivation of factor Va and factor VIIIa by limited proteolysis. The inactivation of factors Va and VIIIa by APC is dependent upon the presence of acidic phospholipids and calcium ions. Protein S has been reported to regulate this activity by accelerating the APC-catalyzed proteolysis of factor Va (Walker, <u>J. Biol. Chem.</u> <u>255</u>:5521-5524 , 1980).

Protein C has also been implicated in the action of tissue-type plasminogen activator (Kisiel and Fujikawa, <u>Behring Inst. Mitt.</u> <u>73</u>:29-42, 1983). Infusion of bovine APC into dogs results in increased plasminogen activator activity (Comp and Esmon, <u>J. Clin. Invest.</u> <u>68</u>:1221-1228, 1981). Other studies (Sakata et al., <u>Proc. Natl. Acad. Sci. USA</u> <u>82</u>:1121-1125, 1985) have shown that addition of APC to cultured endothelial cells leads to a rapid, dose-dependent increase in fibrinolytic activity in the conditioned media, reflecting increases in the activity of both urokinase-related and tissue-type plasminogen activators. APC treatment also results in a dose-dependent decrease in anti-activator activity.

Experimental evidence indicates that activated protein C may be clinically useful in the treatment of thrombosis. The use of APC bypasses the need for <u>in vivo</u> activation of protein C, thus providing a faster acting therapeutic agent.

In addition, exogenous activated protein C has been shown to prevent the coagulopathic and lethal effects of gram negative septicemia (Taylor et al., <u>J. Clin. Invest.</u> <u>79</u> :918-925, 1987). Data obtained from studies with baboons suggest that activated protein C plays a natural role in protecting against septicemia.

Protein C may be purified from clotting factor concentrates (Marlar et al., <u>Blood</u> <u>59</u>:1067-1072, 1982) 5 or from plasma (Kisiel, <u>J. Clin. Invest.</u> <u>64</u>:761-769, 1979) and activated <u>in vitro</u>, but this is a complex and expensive process, and the resulting product may be contaminated with such infectious agents as hepatitis virus, cytomegalovirus, or human immunodeficiency virus (HIV). More recently, methods for producing activated protein C through recombinant DNA technology have been described. Foster et al. (published European Patent Application EP 215,548) disclose the production of activated protein C through the use of cultured mammalian cells transfected with a protein C DNA sequence from which the coding sequence for the activation peptide has been deleted. Foster et al. (EP 266,190) disclose the production of recombinant activated protein C using a DNA sequence encoding an APC precursor with a modified cleavage site.

Furthermore, Bang et al. (EP 319 312) disclose a method for producing activated human protein C by culturing mammalian cells transformed with an expression vector encoding activated protein C in a culture medium containing 10 % serum.

Despite the advances in activated protein C production made possible by the use of genetic engineering, yields remain low and the protein is subject to degradation and/or inactivation during the production process. Thus, there remains a need in the art for methods that enable the production of intact, biologically active activated protein C at higher levels.

Disclosure of Invention

Briefly stated, the present invention discloses methods for producing activated protein C. The methods generally comprise culturing mammalian cells stably transfected with an expression vector comprising a transcriptional promoter operably linked to a DNA sequence encoding activated protein C in a culture medium, wherein said medium contains not more than 0.1% serum, and isolating the activated protein C produced by the cells. In one embodiment, the medium is essentially free of serum.

In one aspect, the DNA sequence further codes for the amino acid sequence $R_1-R_2-R_3-R_4-X-R_5-R_6-R_7-R_6$ between the light and heavy chains of the activated protein C, wherein each of $R_1-R_6$ is lysine or arginine and X is a peptide bond or spacer peptide of 1-12 amino acids.

In another aspect, the DNA sequence further codes for the amino acid sequence $(R_1)_n-R_2-R_3-R_4$, wherein each of $R_1$, $R_2$, $R_3$ and $R_4$ is Lys or Arg and $n = 0, 1, 2$ or $3$, between the light and heavy chains of the activated protein C.

In still another aspect the cells are further transfected to express the <u>Saccharomyces</u> <u>cerevisiae</u> <u>KEX2</u> gene.

Other aspects of the invention will become evident upon reference to the following detailed description and attached drawings.

Brief Description of the Drawings

Figure 1 illustrates the nucleotide sequence of the complete human protein C cDNA and the deduced amino acid sequence of protein C. Arrows indicate cleavage sites for removal of the connecting dipeptide and activation peptide.

Figure 2 illustrates the protein C expression vector p594. Symbols used are 0-1, the adenovirus 5 origin of replication; E, the SV40 enhancer; MLP, the adenovirus 2 major late promoter; L1-3, the adenovirus 2 tripartite leader; 5', 5' splice site; 3', 3' splice site; p(A), polyadenylation signal.

Figure 3 illustrates the construction of plasmids containing the <u>S</u>. <u>cerevisiae</u> <u>KEX2</u> gene.

Figure 4 illustrates gel electrophoresis of recombinant activated protein C from cells grown in the presence (lanes 5-7) or absence (lanes 9-11) of serum. Lane 2, molecular weight markers.

Figure 5 illustrates the plasmids pZMB-1 and pZMB-2. Symbols used include neo, the neomycin resistance gene; SV40 term, SV40 terminator; SV40 prom, SV40 promoter. Other symbols are used as in Figure 2.

Figure 6 illustrates the construction of plasmid pPC1645/229R. DHFR indicates the dihydrofolate reductase gene; MT-1 indicates the mouse metallothionein-1 promoter. Other symbols are used as in Figures 2 and 5.

Best Mode for Carrying Out the Invention

Prior to setting forth the invention, it may be helpful to an understanding thereof to set forth definitions of certain terms to be used hereinafter.

<u>Biological Activity</u>: A function or set of functions performed by a molecule in a biological context (i.e., in an organism or an <u>in</u> <u>vitro</u> facsimile thereof). Biological activities of proteins may be divided into catalytic and effector activities. Catalytic activities of vitamin K-dependent plasma proteins generally involve specific proteolytic cleavages of other plasma proteins, resulting in activation or deactivation of the substrates. Effector activities include specific binding of the biologically active molecules to calcium, phospholipids or other small molecules, to macromolecules, such as proteins, or to cells. Effector activity frequently augments, or is essential to, catalytic activity under physiological conditions.

For activated protein C, biological activity is characterized by its anticoagulant properties. Activated protein C inactivates factor Va and factor VIIIa in the presence of acidic phospholipids and calcium. Protein S appears to be involved in the regulation of this function (Walker, ibid.). The catalytic activities of activated protein C reside in the heavy chain.

<u>Expression Vector</u>: A DNA molecule that contains, <u>inter</u> <u>alia</u>, a DNA sequence encoding a protein of interest (or an insertion site for such a sequence) together with a promoter and other sequences that facilitate expression of the protein. Expression vectors further contain genetic information that provides for their replication in a host cell, either by autonomous replication or by integration into the host genome. Examples of expression vectors commonly used for recombinant DNA. are plasmids and certain viruses, although they may contain elements of both. They also may include a selectable marker.

Stably transfected: The condition in which exogenous DNA has been introduced into cells, and the cells are capable of expressing the exogenous DNA and passing it on to their progeny. Stable transfection is generally achieved by transfecting cells with a selectable marker (e.g. a drug resistance gene) and applying selective pressure. A population of stably transfected cells is clonally identical, being derived from a single transfected progenitor cell. The exogenous DNA may be integrated into the chromosome of the stably transfected cell or may be maintained extrachromosomally. In contrast, cells transiently expressing exogenous DNA are a mixed population including cells which have not taken up the DNA and cells not capable of passing the DNA on to their progeny.

The present invention provides methods of producing a protein that is gamma-carboxylated and has the biological activity of activated protein C through the use of cultured mammalian cells transfected to express the protein. The cells are transfected with an expression vector comprising a promoter operably linked to a DNA sequence encoding activated protein C. The transfected cells are cultured in a medium that has been prepared so as to contain a minimal amount of serum or to be essentially serum-free, and activated protein C is isolated from the medium.

Cloned DNA sequences encoding human protein C have been described (Foster and Davie, Proc. Natl. Acad. Sci. USA 81:4766-4770 , 1984; Foster et al., Proc. Natl. Acad. Sci. USA 82:4673-4677, 1985 and Bang et al., U.S. Pat. No. 4,775,624). A cDNA encoding bovine protein C has been described by Long et al., Proc. Natl. Acad. Sci. USA 81: 5653-5656, 1984. In general, cDNA sequences are preferred for use within the present invention due to their lack of intervening sequences which can lead to aberrant RNA processing and reduced expression levels. Complementary DNAs encoding protein C may be obtained from libraries prepared from liver cells according to standard laboratory procedures. It will be understood, however, that suitable DNA sequences can also be obtained from genomic clones or can be synthesized de novo according to conventional procedures. If partial clones are obtained, it is necessary to join them in proper reading frame to produce a full length clone, using such techniques as endonuclease cleavage, ligation, and loop-out mutagenesis.

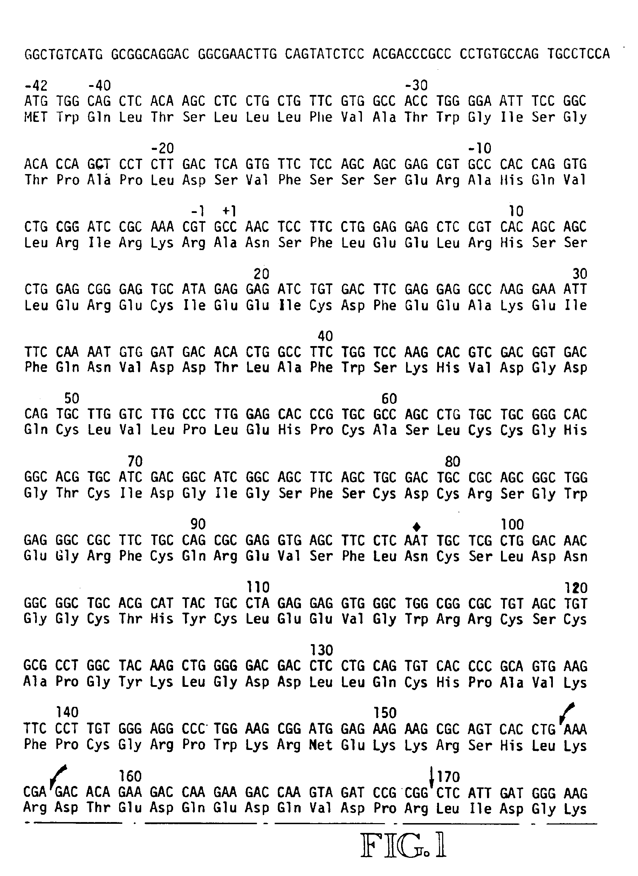

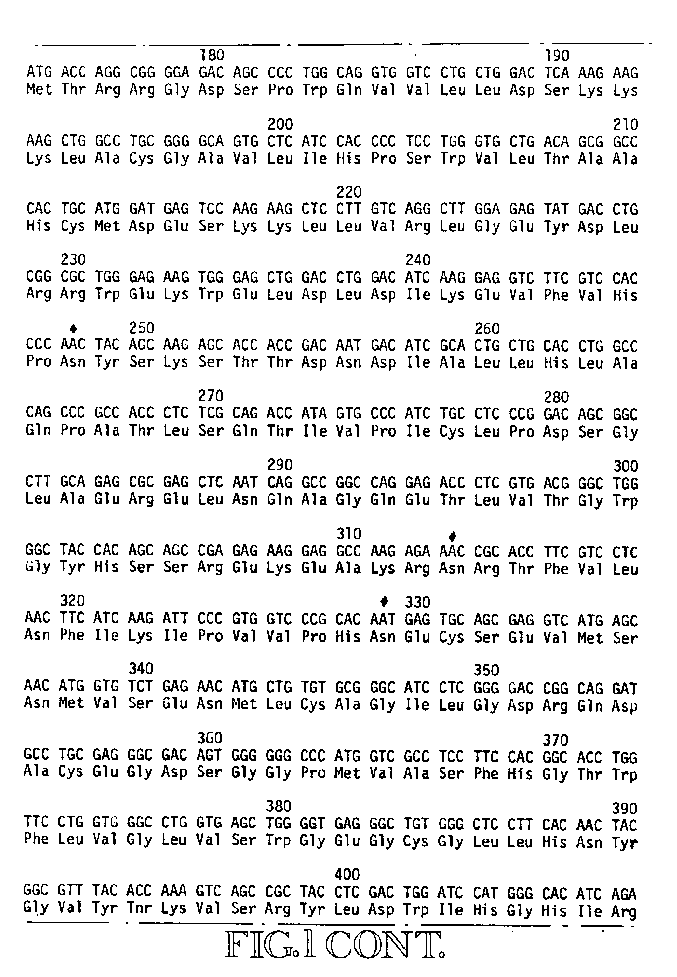

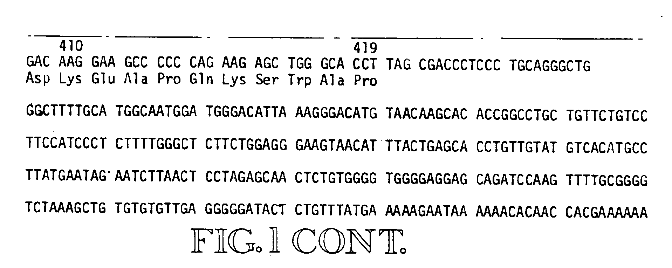

To produce activated protein C, the cloned DNA sequence is modified to delete or replace that portion encoding the activation peptide. The resulting DNA sequence will encode a pre-pro peptide, the light chain of protein C, a processing site and the heavy chain of activated protein C. The DNA sequence may further encode a spacer peptide between the light and heavy chains. In one embodiment, the resultant sequence will encode the light and heavy chains of protein C joined by the sequence Lys-Arg. As used herein, the light chain of activated protein C is understood to comprise amino acids 1-149 of the sequence disclosed in Figure 1 or sequences substantially homologous thereto, or such sequences having C-terminal extensions. The heavy chain of activated protein C is understood not to include the activation peptide (i.e. to begin at animo acid number 170, leucine, as shown in Figure 1). In a preferred embodiment, the DNA sequence is further modified to include a novel cleavage site between the light and heavy chains. The cleavage site may be in the form of the amino acid sequence $(R_1)_n$-$R_2$-$R_3$-$R_4$, wherein $R_1$ through $R_4$ are lysine (Lys) or arginine (Arg) and n is an integer between 0 and 3. Particularly preferred sequences include Arg-Arg-Lys-Arg, Lys-Arg-Lys-Arg and Lys-Lys-Arg. Alternatively, the cleavage site may be of the form $R_1$-$R_2$-$R_3$-$R_4$-X-$R_5$-$R_6$-$R_7$-$R_8$, wherein each of $R_1$ through $R_8$ is Lys or Arg and X is a peptide bond or a spacer peptide of 1 to 12 amino acids. Spacer peptides useful in this regard include the amino acid sequences Asp-Thr-Glu-Asp-Gln-Glu-Asp-Gln-Val-Asp-Pro, Asp-Thr-Glu-Asp-Gln-Glu-Asp-Gln, Asp-Thr-Asp-Gln, Asp-Gln, Asn-Ile-Leu-Asn, and the native protein C activation peptide having the amino acid sequence Asp-Thr-Glu-Asp-Gln-Glu-Asp-Gln-Val-Asp-Pro-Arg. A third group of cleavage site modifications includes the substitution of amino acid residue 154 (His) of native protein C with an amino acid residue selected from the group consisting of Lys, Arg and Leu to give a processing site sequence of the general formula Y-Z-$R_1$-$R_2$, wherein Y is Lys, Arg or Leu; $R_1$ and $R_2$ are Lys or Arg; and Z is an amino acid other than Lys or Arg, preferably Leu.

Modification of the DNA sequence may be obtained by site-specific mutagenesis. Techniques of site-specific mutagenesis are well known in the art and are described by, for example, Zoller and Smith (DNA 3:479-488, 1984). Alternatively, the wild-type protein C sequence may be enzymatically cleaved to remove the native activation peptide sequence, and the sequences encoding the heavy and light chains joined to a synthesized activation peptide containing one of the cleavage sites described above.

As will be understood by those skilled in the art, the methods of the present invention can also be used to produce variants and analogs of activated protein C. Variants and analogs of activated protein C include those containing minor amino acid changes, such as those due to genetic polymorphism, and those in which amino acids have been added, deleted or replaced without substantially altering the biological activity of the protein. Activated protein C analogs further include proteins that have the protein C amino-terminal portion (gla domain) substituted with a gla domain of one of the vitamin-K dependent plasma proteins factor VII, factor IX, factor X, prothrombin or protein S.

As noted above, DNA sequences for use within the present invention will encode a pre-pro peptide at the amino-terminus of the activated protein C precursor in order to obtain proper post-translational processing (e.g. gamma-carboxylation of glutamic acid residues) and secretion from the host cell. The pre-pro peptide may be that of protein C or another vitamin K-dependent plasma protein, such as factor VII, factor IX, factor X, prothrombin or protein S.

The DNA sequence encoding activated protein C is then inserted into a suitable expression vector, which is in turn used to transfect cultured mammalian cells. Expression vectors for use in carrying out the present invention will comprise a promoter capable of directing the transcription of a cloned gene or cDNA. Preferred promoters include viral promoters and cellular promoters. Viral promoters include the SV40 promoter (Subramani et al., Mol. Cell. Biol. 1:854-864, 1981) and the CMV promoter (Boshart et al., Cell 41:521-530, 1985). A particularly preferred viral promoter is the major late promoter from adenovirus 2 (Kaufman and Sharp, Mol. Cell. Biol. 2: 1304-13199, 1982). Cellular promoters include the mouse kappa gene promoter (Bergman et al., Proc. Natl. Acad. Sci. USA 81:7041-7045, 1983) and the mouse $V_H$ promoter (Loh et al., Cell 33:85-93, 1983). A particularly preferred cellular promoter is the metallothionein-I promoter (Palmiter et al., Science 222:809-814, 1983). Expression vectors may also contain a set of RNA splice sites located downstream from the promoter and upstream from the insertion site for the activated protein C sequence or within the activated protein C sequence itself. Preferred RNA splice sites may be obtained from adenovirus and/or immunoglobulin genes. Also contained in the expression vectors is a polyadenylation signal located downstream of the insertion site. Particularly preferred polyadenylation signals include the early or late polyadenylation signal from SV40 (Kaufman and Sharp, ibid.), the polyadenylation signal from the adenovirus 5 Elb region or the human growth hormone gene terminator (DeNoto et al. Nuc. Acids Res. 9:3719-3730, 1981). The expression vectors may also include a noncoding viral leader sequence, such as the adenovirus 2 tripartite leader, located between the promoter and the RNA splice sites; and enhancer sequences, such as the SV40 enhancer and the sequences encoding the adenovirus VA RNAs.

Cloned DNA sequences are introduced into cultured mammalian cells by, for example, calcium phosphate-mediated transfection (Wigler et al., Cell 14:725-732, 1978; Corsaro and Pearson, Somatic Cell Genetics 7:603-616, 1981; Graham and Van der Eb, Virology 52:456-467, 1973) or electroporation (Neumann et al., EMBO J. 1:841-845, 1982). In order to identify and select for cells that express the exogenous DNA, a gene that confers a selectable phenotype (a selectable marker) is generally introduced into the cells along with the gene or cDNA of interest. Preferred selectable markers include genes that confer resistance to drugs such as neomycin, hygromycin, and methotrexate. The selectable marker may be an amplifiable selectable marker. A preferred amplifiable selectable marker is a dihydrofolate reductase (DHFR) sequence. A particularly preferred amplifiable marker is the DHFR$^r$ cDNA (Simonsen and Levinson, Proc. Natl. Acad. Sci. USA 80:2495-2499, 1983). Selectable markers are reviewed by Thilly (Mammalian Cell Technology, Butterworth Publishers, Stoneham, MA), and the choice of selectable markers is well within the level of ordinary skill in the art.

Selectable markers may be introduced into the cell on a separate plasmid at the same time as the gene of interest, or they may be introduced on the same plasmid. If on the same plasmid, the selectable marker and the gene of interest may be under the control of different promoters or the same promoter, the latter arrangement producing a dicistronic message. Constructs of this type are known in the art (for example, Levinson and Simonsen, U.S. Patent 4,713,339). It may also be advantageous to add additional DNA, known as "carrier DNA," to the mixture that is introduced into the cells.

After the cells have taken up the DNA, they are grown in an appropriate growth medium, typically 1-2 days, to begin expressing the gene of interest. As used herein the term "appropriate growth medium" means a medium containing nutrients and other components required for the growth of cells. Media generally include a carbon source, a nitrogen source, essential amino acids, essential sugars, vitamins, salts, phospholipids, protein and growth factors. Drug selection is then applied to select for the growth of cells that are expressing the selectable marker in a stable fashion. For cells that have been transfected with an amplifiable selectable marker the drug concentration may be increased in a stepwise manner to select for an increased copy number of the clcned sequences, thereby increasing expression levels. Clones of stably transfected cells are then screened for expression of activated protein C.

Preferred mammalian cell lines for use in the present invention include the COS-1 (ATCC CRL 1650), BHK and 293 (ATCC CRL 1573; Graham et al., J. Gen. Virol. 36:59-72, 1977) cell lines. A preferred BHK cell line is the tk⁻ts13 BHK cell line (Waechter and Baserga, Proc. Natl. Acad. Sci USA 79: 1106-1110, 1982). In addition, a number of other cell lines may be used within the present invention, including Rat Hep I (ATCC CRL 1600), Rat Hep II (ATCC CRL 1548), TCMK (ATCC CCL 139), Human lung (ATCC CCL 75.1), Human hepatoma (ATCC HTB-52), Hep G2 (ATCC HB 8065), NCTC 1469 (ATCC CCL 9.1) and DUKX cells (Urlaub and Chasin, Proc. Natl. Acad. Sci. USA 77:4216-4220, 1980).

Processing of activated protein C precursors by cleavage after a Lys-Arg dipeptide between the light and heavy chains may be enhanced by introducing the S. cerevisiae KEX2 gene into the host cell. The KEX2 gene encodes an endopeptidase that cleaves after a dibasic amino acid sequence (Fuller et al., in Leive, ed., <u>Microbiology: 1986</u>, 273-278, 1986). A cultured mammalian cell line stably transfected with this gene is thus useful for expressing activated protein C.

In a preferred embodiment, mammalian cells that have been stably transfected to express activated protein C are grown for a period of time, preferably to confluency, in serum-containing media (e.g. media containing from about 1% to about 10% serum), then transferred to medium that has been formulated to contain not more than 0.1% serum. The inventors have discovered that reducing or eliminating serum from the media increases the yield of activated protein C from stably transfected cells. A variety of serum-free cell culture media are known in the art. (See, for example, Barnes and Sato, <u>Cell</u> <u>22</u>: 649-656, 1980; Barnes, <u>Biotechniques</u> <u>5</u>: 534-542, 1987; and catalogs of the American Type Culture Collection, Rockville, MD.) Culture media are also available from a number of commercial suppliers. A particularly preferred culture medium in this regard is a mixture of 50% Dulbecco's modified Eagle's Medium (DMEM) and 50% Ham's F12 containing 1 mM sodium pyruvate, 2mM L-glutamine, 50 mg/l penicillin, 50 mg/l streptomycin, 100 mg/l neomycin, 5 mg/l insulin, 3 μg/l selenium, 10 mg/l fetuin, 20 mg/l transferrin and 25 mM pH 7.2 HEPES buffer. The transferrin can be replaced with bovine serum albumin (1 g/l) or meat hydrolysate (e.g. Primatone RL®, Sheffield Products, Norwich, NY; 2.5 g/l). The medium may also contain serum, preferably fetal bovine serum, at up to 0.1% It is preferred that the culture medium also contain vitamin K to facilitate the formation of gamma-carboxyglutamic acid residues. Concentrations of vitamin K in the range of 5 ng/ml to 5 μg/ml are sufficient, with 1 μg/ml preferred. The cells are maintained in the minimal (</=0.1%) serum or serum-free medium for up to about seven days, during which time the medium is harvested and the activated protein C is isolated. The cells are then returned to media containing a high level of serum and allowed to resume growing. As will be appreciated by those skilled in the art, the cells may alternatively be grown in serum-free media without the need for initial or subsequent growth in the presence of serum.

The cells are cultured under conditions generally used in the art. In this regard it is preferred to culture the cells at between 36°C and 40°C under conditions which maintain a pH between about 6.8 and 8.0, preferable about pH 7.2. The pH may be maintained through the use of a variety of buffer systems known in the art. A preferred buffer system involves culturing the cells in a bicarbonate buffer in a humidified incubator containing $CO_2$, preferably about 5% $CO_2$.

Activated protein C produced according to the present invention may be purified by affinity chromotography on an anti-protein C antibody column. The use of calcium-dependent monoclonal antibodies, as described by Wakabayashi et al. (<u>J. Biol. Chem.</u> <u>261</u>:11097-11108, 1986), is particularly preferred. Additional purification may be achieved by conventional chemical purification means, such as high-performance liquid chromatography (HPLC). Other methods of purification, including barium citrate precipitation, are known in the art and may be applied to the purification of recombinant activated protein C.

The activated protein C produced according to the present invention may be used in pharmaceutical compositions for topical or intravenous application, generally in combination with a physiologically acceptable carrier or diluent. Preferred carriers and diluents include saline and sterile water. Pharmaceutical compositions may also contain stabilizers and adjuvants. The resulting aqueous solutions may be packaged for use or filtered under aseptic conditions and lyophilized, the lyophilized preparation being combined with a sterile aqueous solution prior to administration.

The following examples are offered by way of illustration and not by way of limitation.

<u>EXAMPLES</u>

Restriction endonucleases and other DNA modification enzymes (e.g., T4 polynucleotide kinase, 5 calf alkaline phosphatase, DNA polymerase I (Klenow fragment), T4 polynucleotide ligase) were obtained from Bethesda Research Laboratories (BRL) and New England Biolabs and were used as directed by the manufacturer, unless otherwise noted.

Oligonucleotides were synthesized on an Applied Biosystems Model 380A DNA synthesizer and purified by polyacrylamide gel electrophoresis on denaturing gels. <u>E. coli</u> cells were transformed as described by Maniatis et al. (<u>Molecular Cloning: A</u> <u>Laboratory Manual</u>, Cold Spring Harbor Laboratory, 1982). M13 and pUC cloning vectors and host strains were obtained from BRL.

<u>EXAMPLE 1</u>

<u>Cloning of DNA Sequences Encoding Human Protein C</u>

A cDNA coding for a portion of human protein C was prepared as described by Foster and Davie (ibid.).

Briefly, a λgt11 cDNA library was prepared from human liver mRNA by conventional methods. Clones were screened using an $^{125}$I-labeled affinity-purified antibody to human protein C, and phage were prepared from positive clones by the plate lysate method (Maniatis et al., ibid.), followed by banding on a cesium chloride gradient. The cDNA inserts were removed using Eco RI and were subcloned into plasmid pUC9 (Vieira and Messing, Gene 19:259-268, 1982). Restriction fragments were subcloned in the phage vectors M13mp10 and M13mp11 (Messing, Meth. in Enzymology 101:20-77, 1983) and were sequenced by the dideoxy method (Sanger et al., Proc. Natl. Acad. Sci. USA 74:5463-5467, 1977). A clone that contained DNA corresponding to the known partial sequence of human protein C (Kisiel, ibid., 1979) and encoded protein C beginning at amino acid 64 of the light chain and extending through the heavy chain and into the 3' non-coding region was selected. This clone was designated λHC1375, A second cDNA clone coding for protein C from amino acid 24 was also identified. The insert from the larger clone was subcloned into pUC9 and the plasmid was designated pHCλ6L. This clone encodes a major portion of protein C, including the heavy chain coding region, termination codon, and 3' non-coding region.

The cDNA insert from λHC1375 was nick translated using $\alpha$-$^{32}$P dNTP's and used to probe a human genomic library in phage λCharon 4A (Maniatis et al., Cell 15:687-702, 1978) using the plaque hybridization procedure of Benton and Davis (Science 196:181-182, 1977) as modified by Woo (Meth. Enzymol. 68:381-395, 1979). Positive clones were isolated and plaque-purified (Foster et al., Proc. Natl. Acad. Sci. USA 82:4673-4677, 1985, herein incorporated by reference). Phage DNA prepared from positive clones (Silhavy et al., in Experiments with Gene Fusion, Cold Spring Harbor Laboratory, 1984) was digested with Eco RI or Bgl II and the genomic inserts were purified and subcloned in pUC9. Restriction fragments of the genomic inserts were subcloned into M13 vectors and sequenced to confirm their identity and establish the DNA sequence of the entire gene.

The cDNA insert of pHCλ6L was nick translated and used to probe the phage λCharon 4A library. One genomic clone was identified that hybridized to probes made from the 5' and 3' ends of the cDNA. This phage clone was digested with Eco RI, and a 4.4 kb fragment, corresponding to the 5' end of the protein C gene, was subcloned into pUC9. The resultant recombinant plasmid was designated pHCR4.4. Complete DNA sequence analysis revealed that the insert in pHCR4.4 included two exons of 70 and 167 base pairs separated by an intron of 1263 bp. The first exon encodes amino acids -42 to -19; the second encodes amino acids -19 to 37. Sequence analysis confirmed the DNA sequence of the entire protein C gene.

A genomic fragment containing an exon corresponding to amino acids -42 to -19 of the pre-pro peptide of protein C was isolated, nick translated, and used as a probe to screen a cDNA library constructed by the technique of Gubler and Hoffman (Gene 25:263-269, 1983) using mRNA from Hep G2 cells. This cell line was derived from human hepatocytes and was previously shown to synthesize protein C (Fair and Bahnak, Blood 64: 194-204, 1984). Ten positive clones comprising cDNA inserted into the Eco RI site of phage λgt11 were isolated and screened with an oligonucleotide probe corresponding to the 5' non-coding region of the protein C gene. One clone was also positive with this probe and its entire nucleotide sequence was determined. The cDNA contained 70 bp of 5' untranslated sequence, the entire coding sequence for human pre-pro-protein C, and the entire 3' non-coding region corresponding to the second polyadenylation site. The cDNA sequence and deduced amino acid sequence are shown in Figure 1.

## EXAMPLE 2

## Expression of Activated Protein C

### A. Construction and Expression of pPC829.

The cDNA sequence encoding protein C was altered by site-specific mutagenesis to delete the portion encoding the activation peptide. The amino acid sequence of the junction between the light and heavy chains of the encoded APC precursor, designated 829, is shown in Table 1. The altered sequence was then transfected into tk⁻ts13 BHK and 293 cells, and stably transfected cells were selected. Active protein C was detected in culture media samples from both cell lines.

## Table 1

### Amino Acid Sequences of Cleavage-Site Mutants

594WT

```
   149                    155                                              170
   E-K  -K-R-S-H-L-      K-R-D-T-E-D-Q-E-D-Q-V-D-P-R-L-I-D-
829
   E-K-  K-R-S-H-L-      K-R-                              L-I-D-
962
   E-K-  K-R-S-H-L-R-R-K-R-D-T-E-D-Q-E-D-Q-V-D-P-R-L-I-D-
1058
   E-K-  K-R-S-H-L-R-R-K-R-                              L-I-D-
1645
   E-K-  K-R-S-H-L-R-R-K-R-D-T-E-D-Q-E-D-Q-R-R-K-R-L-I-D-
1880
   E-K-  K-R-S-H-L-R-R-K-R-D-T-     D-Q-R-R-K-R-L-I-D-
1953
   E-K-  K-R-S-H-L-R-R-K-R-         R-R-K-R-L-I-D-
1954
   E-K-  K-R-S-H-L-R-R-K-R-D-       Q-R-R-K-R-L-I-D-
1962
   E-K-  K-R-                                          L-I-D-
2043
   E-K-R-K-R-                                          L-I-D-
```

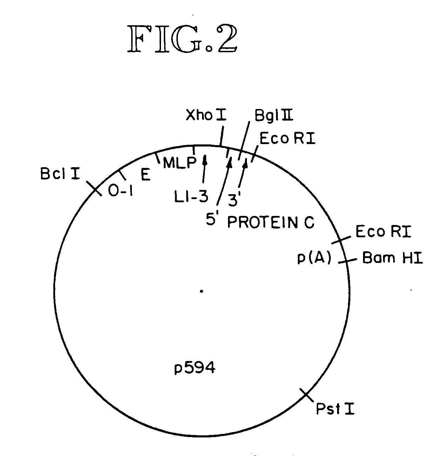

The protein C cDNA was isolated as an Eco RI fragment and cloned into the vector pDX as disclosed in published European Patent Application EP 266,190. Recombinant plasmids were screened by restriction analysis to identify those having the protein C insert in the correct orientation with respect to the promoter elements, and plasmid DNA (designated pDX/PC) was prepared from a correct clone. Because the cDNA insert in pDX/PC contained an ATG codon in the 5' non-coding region (see Figure 1), oligonucleotide-directed deletion mutagenesis was performed on the cDNA to remove the three base pairs. The resulting vector, designated p594, contains the protein C cDNA operably linked to the adenovirus 2 major late promoter (Figure 2). This vector also contains the adenovirus 5 origin of replication (0-1 map units sequence), the SV40 enhancer, the adenovirus 2 tripartite leader, a set of RNA splice sites, an SV40 polyadenylation signal and a dihydrofolate reductase gene as a selectable marker.

To delete the activation peptide coding sequence, plasmid p594 was digested with Sst I, and the ~880 bp fragment was purified and inserted into the Sst-I site of M13mp10 (Messing, Methods Enzymol. 101: 20-78, 1983). The 12 activation peptide codons were deleted by oligonucleotide-directed deletion mutagenesis (Zoller and Smith, DNA 3:479-488, 1984) using the mutagenic oligonucleotide ZC829 (5' CTG AAA CGA CTC ATT GAT 3'). Replicative form DNA was prepared from mutant phage clones and digested with Sst I. The protein C fragment (~840 bp) was isolated and inserted into Sst I-digested p594. The resultant plasmids were screened for proper orientation of the Sst I fragment by restriction mapping using Bgl II. A correct plasmid was selected and designated pPC829. Plasmid pPC829 was sequenced to verify the presence of the desired coding sequence.

Plasmid pPC829 was co-transfected into tk⁻ts13 BHK cells (with plasmid pSVDHFRT (Lee et al., Nature 294:228-232, 1982)) and 293 cells (with pKO-neo) by calcium phosphate coprecipitation (Graham and van der Eb, Virology 52:456-467, 1973). After 48 hours, culture media were harvested and assayed for protein C by enzyme-linked immunosorbent assay (ELISA) using the affinity-purified polyclonal antibody that was used in the initial identification of the cDNA clones and/or a monoclonal antibody directed against the heavy chain of protein C. The affinity-purified antibody to human protein C (100 µg/ml in 0.1 M $Na_2CO_3$, pH 9.6) was added to each well of 96-well microtiter plates, and the plates were incubated overnight at 4°C. The wells were washed three times with PBS (5 mM phosphate buffer, pH 7.5, 0.15 M NaCl) containing 0.05% Tween-20 to remove unbound antibody and were incubated with 100 µl of 1% bovine serum albumin, 0.05% Tween 20 in PBS at 4°C overnight. The plates were rinsed several times with PBS, air dried, and stored at 4°C. To assay samples, 100 µl of each sample was incubated for 1 hour at 37°c in the coated wells, and the wells were rinsed with 0.05% Tween-20 in PBS. The plates were then incubated for 1 hour at 37°C with a biotin-conjugated sheep polyclonal antibody to protein C (30 ng/ml) in PBS containing 1% bovine serum albumin and 0.05% Tween-20. The wells were rinsed with PBS and incubated for 1 hour at 37°C with avidin-conjugated alkaline phosphatase in PBS containing 1% bovine serum albumin and 0.05% Tween-20. The wells were rinsed with PBS, and alkaline phosphatase activity was measured by the addition of 100 µl of phosphatase substrate (Sigma 104; 600 µg/ml in 10% diethanolamine, pH 9.8, containing 0.3 mM $MgCl_2$). The absorbance at 405 nm was read on a microtiter plate reader. Results are shown in Table 2. At the same time, cultures were split 1:5 into media containing 500 µg/ml of G418 (293 cells) or 250 nM methotrexate (tk⁻ ts13 BHK cells).

## TABLE 2

### TRANSIENT EXPRESSION OF ACTIVATED PROTEIN C (ELISA)

| Cell Line | Protein C ng/ml in Media |
|---|---|
| tk⁻ts13 BHK | 2.7 |
| 293 | 30 |

After being grown 10 days in the presence of selective media, stably transfected colonies were screened for activated protein C production by immunofilter assay (McCracken and Brown, BioTechniques, 82-87, March/April 1984). Plates were rinsed with PBS or No Serum medium (DMEM plus 1x PSN antibiotic mix, 5 µg/ml vitamin K). Teflon® mesh (Spectrum Medical Industries, Los Angeles, CA) was then placed over the cells. Nitrocellulose filters were wetted with PBS or No Serum medium, as appropriate, and placed over the mesh. After a four hour incubation at 37°C, the filters were removed and placed in filter buffer (50 mM Tris pH 7.4, 5 mM EDTA, 0.05% NP-40, 150 mM NaCl, 0.25% gelatin) for 30 minutes at room temperature. The filters were incubated for 1 hour at room temperature, with shaking, in biotin-labeled sheep anti-protein C polyclonal antibody (1 µg/ml in filter buffer). Filters were then washed in the same buffer and incubated 1 hour at room temperature, with shaking, in avidin-conjugated horseradish peroxidase (Boehringer-Mannheim) (diluted 1:1000 in the filter buffer). Filters were washed in 50 mM Tris-HCl, pH 7.4, 5 mM EDTA, 1 M NaCl, 0.25% gelatin, 0.4% sacrosyl, 0.05% NP-40, then in $H_2O$. The washed filters were incubated in color reagent (60 mg HRP color development reagent [Bio-Rad], 20 ml methanol, 100 µl $H_2O_2$ in 100 ml 50 mM Tris pH 7.4, 150 mM NaCl). The reaction was stopped by transferring the filters to $H_2O$.

Positive colonies were picked and grown in selective media (containing 500 µg/ml G418 or 250 nM methotrexate, as appropriate) for 10 days. Culture media were assayed for APC activity by chromogenic assay. Media samples were added to microtiter wells containing 100 µl of 0.2 mM Spectrozyme PCa (American Diagnostica #336) in 50 mM Tris pH 7.5, 150 mM NaCl. Plates were incubated at 37°C, and the $A_{405}$ was measured at various time intervals. Media from positive 293 cell colonies consistently showed higher activity with the chromogenic substrate for APC than did control media which had been incubated with non-transfected 293 cells for the same length of time (10 days).

### B. Construction and Expression of pDX/PC1058.

A DNA sequence encoding an activated protein C precursor with the cleavage site sequence Arg-Arg-Lys-Arg was constructed by mutagenesis of the wild-type protein C sequence. The resultant sequence was designated 1058. The amino acid sequence of this precursor at the junction between the light and heavy chains is presented in Table 1.

The protein C sequence present in plasmid p594 was altered in a single mutagenesis to delete the codons for the activation peptide and insert the Arg-Arg codons at the processing site. Mutagenesis was performed on the 870 bp Sst I fragment from p594, which was cloned into M13mp11. Single-stranded template DNA was isolated and mutagenized using oligonucleotides ZC1058 (5' CGC AGT CAC CTG AGA AGA AAA CGA CTC ATT GAT GGG 3') and ZC550 (5' TCC CAG TCA CGA CGT 3').

The mutagenized sequence was isolated from replicative form DNA as an Sst I fragment. The mutagenized fragment was joined to Sst I-cut p594 to constuct expression vector pDX/PC1058. The vector was co-transfected into tk⁻ts13 BHK cells with pSV2-DHFR (Subramani et al., Mol. Cell. Biol. 1:854-864, 1981) by the calcium phosphate procedure (essentially as described by Graham and van der Eb, ibid.). The transfected cells were grown in Dulbecco's modified Eagle's medium (DMEM) containing 10% fetal calf serum, 1x PSN antibiotic mix (Gibco 600-5640), 2.0 mM L-glutamine and vitamin K (5 $\mu$g/ml). The cells were selected in 250 nM methotrexate (MTX) for 14 days. The protein was purified by affinity chromatography on a column prepared by coupling 7 mg of polyclonal sheep antibody against human protein C to 2 grams of CNBr-activated Sepharose 4B (Pharmacia Inc., Piscataway, NJ). Cell culture medium was applied to the column and the column was washed with 100 ml TBS (50 mM Tris pH 7.5, 150 (mM)M Nacl). The activated protein C was eluted with TBS containing 3 M KSCN or with pH 11.5 buffer (25 mM potassium phosphate, pH 11.5, 0.2 M NaCl, 2% Tween-80, 0.5% NaN$_3$).

### C. Expression of Activated Protein C from pDX/PC1058 in a KEX2 Transfected Cell Line

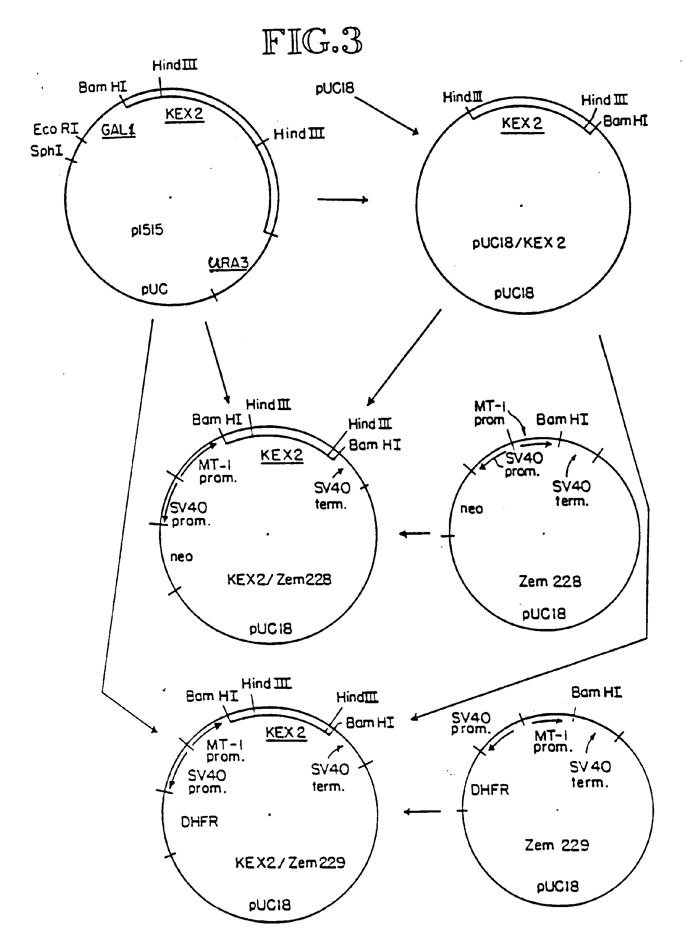

The Saccharomyces cerevisiae KEX2 gene was isolated from a yeast genomic library by screening transformed kex2 mutant cells for production of an $\alpha$-factor halo on a lawn of a suitable tester cells. One clone was obtained that complemented all reported defects of kex2 mutants (mating, $\alpha$-factor production, maturation of killer toxin and sporulation in a homozygous diploid strain). The gene was subcloned into a pUC vector under the control of the yeast GAL1 promoter. The resultant plasmid, designated p1515, has been deposited with American Type Culture Collection under accession number 67569. As shown in Figure 3, p1515 was digested with Hind III, and a 2.1 kb fragment was recovered. This fragment was ligated to Hind III-cut pUC18 to construct plasmid pUC18/KEX2. The KEX2 fragment (2.1 kb) was then isolated from pUC18/KEX2 by digesting the plasmid partially with Hind III and to completion with Bam HI. The remainder of the KEX2 sequence was then isolated as a 0.43 kb fragment from a Bam HI + Hind III digest of p1515. The two KEX2 fragments were then ligated into the Bam HI site of the vectors Zem228 and Zem229. (Zem229 is a pUC18-based expression vector that contains a unique Bam HI site for insertion of cloned DNA between the mouse metallothionein-I promoter and the SV40 transcription terminator. This vector also contains an expression unit comprising the SV40 early promoter, mouse dihydrofolate reductase gene and SV40 terminator. Zem228 is similar to Zem229 but contains a neomycin resistance gene in place of the DHFR gene. Thus, in Zem228 the inserted gene is under the control of the metallothionein-I promoter and SV40 terminator, and the vector can be selected with the antibiotic G418.) The resulting plasmids were designated KEX2/Zem228 and KEX2/Zem229, respectively

A high protein C producing pDX/PC1058-transfected tk⁻ts13 BHK clone (pDX/PC1058-3//BHK) was transfected with KEX2/Zem228 by the calcium phosphate procedure. Transfected cells were selected with 500 $\mu$g/ml G418 and 250 nM methotrexate.

A selected clone, designated KEX2-1058//BHK, was pulse-labeled with $^{35}$S-cysteine in cysteine-free DMEM (Gibco Laboratories, Grand Island, NY) containing 1% fetal calf serum for 24 hours. The culture media were collected and assayed for the presence of single-chain and two-chain protein C by immunoprecipitation with a monoclonal antibody to protein C. Two hundred and fifty $\mu$l of media was combined with 10 $\mu$g of antibody, and the mixture was incubated at 37°C for one hour. One hundred $\mu$l of Staph A cell suspension (Pharmacia, Piscataway, NJ) was added, and the mixture was incubated at 37°C for one hour. The cells were pelleted by centrifugation, and the pellet was resuspended in 60 $\mu$l of gel buffer containing 1% $\beta$-mercaptoethanol. The suspension was heated to 100°C for three minutes, then electrophoresed on an SDS-polyacrylamide gel. Proteins were visualized by autoradiography. The KEX2-1058//BHK clone showed approximately 100% cleavage of the protein into the two-chain form.

A stably transfected BHK clonal cell line designated KEX2-1058 #3-5 was grown at 37°C in a 5% CO$_2$ atmosphere in cell factories (Nunc, Thousand Oaks, CA) to confluency in medium containing 10% fetal bovine serum, then allowed to grow in medium containing 1% serum. The conditioned medium was removed by decanting, and the cells were washed twice with 500 ml PBS. The washed cells were transfered to serum-free medium containing 50% DMEM, 50% Ham's F12, 1 mM sodium pyruvate, 2mM L-glutamine, 1x PSN antibiotic mix (Gibco Laboratories), 5 mg/l insulin, 3 $\mu$g/l selenium, 10 mg/l fetuin, 20 mg/l transferrin and 25 mM pH 7.2 HEPES buffer. All media contained 1 $\mu$g/ml vitamin K. After three to four days of growth the medium was harvested and the cells were transferred to medium containing 1% serum.

Activated protein C was purified from serum-free and serum-containing media by immunoaffinity chromatography on a PCL-2-Sepharose column. This column was constructed by coupling a monoclonal antibody (designated PCL-2) specific for the $Ca^{++}$-bound light chain of protein C to CNBr-activated Sepharose (Pharmacia, Piscataway, NJ). The conditioned media were filtered and concentrated about 90-fold using an Amicon DC10L concentrator (Amicon, Danvers, MA) prior to purification. The concentrated samples were applied to the column in the presence of 10 mM $CaCl_2$. The column was washed with 50 mM Tris-HCl, 1.0 M NaCl, 10 mM $CaCl_2$, pH 7.5. Activated protein C was eluted from the column with 15 mM EDTA in 50 mM Tris-HCl pH 7.5.

Activated protein C produced by cells cultured in serum-free medium and medium containing 1% serum was quantitated using the BCA method (Pierce Chemical Co., Rockford, IL). Biological activity of the protein was determined by following the prolongation of APTT with pooled normal plasma as substrate. Briefly, the APTT assay was carried out by incubating 100 μl of normal plasma with various dilutions (100 μl volume) of recombinant APC at 37°C for 50 seconds, followed by incubation with 100 μl of Actin FS (Dade, Miami, FL) at 37°C for 100 seconds. 100 μl of 25mM $CaCl_2$ was then added, and the clotting time was determined. Results of multiple determinations are shown in Table 3. Values are rounded off to two significant digits.

## TABLE 3

### BIOLOGICAL ACTIVITY OF RECOMBINANT APC

| | Clotting Time (Seconds) | | | | | |
|---|---|---|---|---|---|---|
| | +1% Serum | | | - Serum | | |
| Experiment: | 2 | 3 | 4 | 3 | 4 | 5 |
| APC (λg) | | | | | | |
| 20 | 7.6 | 9.0 | 5.0 | 13 | 15 | 19 |
| 40 | 12 | 15 | 12 | 21 | 28 | 25 |
| 60 | 18 | 21 | 17 | 28 | 33 | 32 |
| 80 | 18 | 20 | 19 | 32 | 37 | 39 |
| 100 | 25 | 23 | 20 | 37 | 41 | 49 |

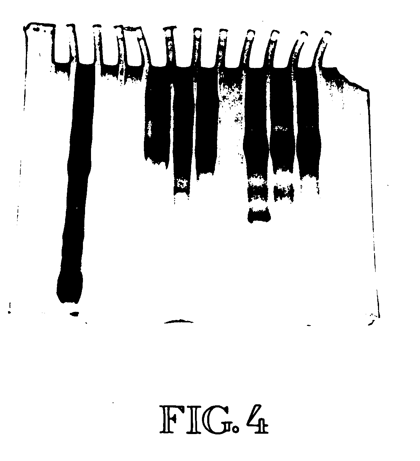

The proteins were also characterized by SDS-polyacrylamide gel electrophoresis (Laemmli, Nature 227: 680, 1970). As shown in Figure 4, recombinant activated protein C produced by cells cultured in serum-free medium is predominantly present as a species with a molecular weight of approximately 68 kDa (lanes 9-11). In contrast, APC from cells cultured in the presence of 1% serum contains significant amounts of a species with a molecular weight greater than 93 kDa (lanes 5-7), suggesting that a high molecular weight APC-inhibitor complex forms in the presence of serum.

These data indicate that production of recombinant activated protein C in serum-free media results in increased yields of intact, biologically active protein as compared to production in the presence of conventional levels of serum.

### D. Expression of Activated Protein C from pPC1962/ZMB-2 in KEX2 Transfected Cells

The coding sequence of protein C was altered to remove amino acids 153-169, resulting in an activated protein C precursor with a light chain-heavy chain junction between amino acids 152 and 170. The sequence of this activated protein C precursor, designated 1962, is presented in Table 1.

Oligonucleotide-directed mutagenesis was carried out on a template comprising the Sst I fragment of p594 inserted, in the proper orientation, into the Sst I site of M13mp10. Single-stranded template DNA was prepared from the 594/mp10 phage clone. Oligonucleotide-directed mutagenesis was carried out on the template using the synthetic oligonucleotides ZC1962 (5' GAG AAG AAG CGC CTC ATT GAT GGG 3') and ZC550. Positive phage clones were sequenced to confirm the mutagenesis. A positive phage clone was designated 1962.

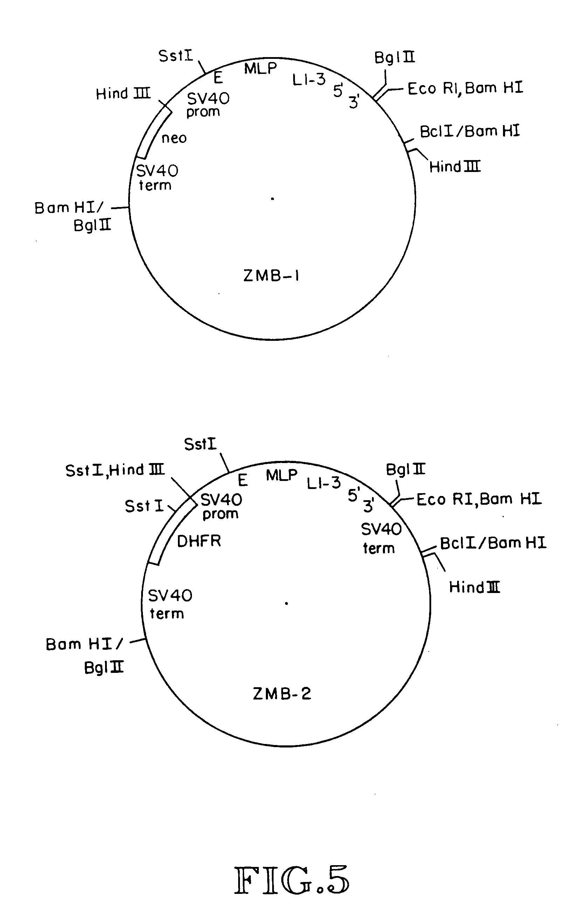

Replicative form DNA was prepared from phage clone 1962 and was digested with Sst I and Pst I to isolate the approximately 0.4 kb mutagenized fragment. Plasmid PC229/962 (Example 2E) was digested with Eco RI and Pst I to isolate the 562 bp protein C fragment. A 700 bp Sst I-Eco RI protein C fragment was obtained from PC1869/229R (a Zem229R-based plasmid comprising the p594 protein C coding sequence, but with the Arg (residue 157) codon substituted with a Lys codon. Plasmid Zem229R is similar to Zem229 except that the Eco RI sites present in Zem299 have been destroyed by partial digestion, blunt ending by treatment with DNA polymerase I (Klenow fragment) and dNTP's followed by religation, and a unique Eco RI site was created at the Bam HI site by digestion with Bam HI and religation with Bam HI-Eco RI adapters). Plasmid pZMB-2 (Figure 5) was linearized by digestion with Eco RI. (Plasmid pZMB-2 is similar to Zem229R but contains the SV40 enhancer, adenovirus 2 major late promoter, adenovirus 2 tripartite leader, and 5' and 3' splice sites substituted for the MT-1 promoter using an Sst I-Hind III adapter.) The approximately 0.4 kb Pst I-Sst I fragment from phage clone 1962, the 700 bp Pst I-Eco RI fragment from pC1869/229R, the 562 bp Sst I-Eco RI fragment from PC229/962 and the linearized pZMB-2 were joined in a four-part ligation. A plasmid with the insert in the correct orientation was designated pPC1962/ZMB-2.

Plasmid pPC1962/ZMB-2 was transfected into tk⁻ ts13 BHK cells by calcium phosphate co-precipitation. Transfected cells were grown in DMEM containing 10% fetal calf serum, 1x PSN antibiotic mix (Gibco), 2.0 mM L-glutamine and 5 μg/ml vitamin K. The cells were selected in 500 nM methotrexate for 15 days, and the resulting colonies were screened by the immunofilter assay. The most intensely reacting colonies were picked by cylinder cloning and were grown individually in 10 cm plates. When the cultures were nearly confluent, protein C production levels were measured by ELISA.

A high protein C producing pPC1962/ZMB-2 transfectant was transfected with KEX2/ZMB-1. (KEX2/ZMB-1 comprises the KEX2 coding sequence inserted into the vector ZMB-1 at the unique Eco RI site. ZMB-1, as shown in Figure 5, is similar to ZMB-2 but was constructed from Zem228R. Zem228R was prepared from Zem228 as described above for the construction of Zem229R.) Co-transfected cells were selected and media samples were collected. Activated protein C was dectected in media samples from pPC1962-KEX2/ZMB-1 co-transfected cells.

E. Construction and Expression of pPC1645/Zem229R.

A DNA sequence encoding an eight amino acid spacer peptide flanked by Arg-Arg-Lys-Arg processing signals at the junction between the light and heavy chains of protein C (designated 1645) is presented in Table 1.

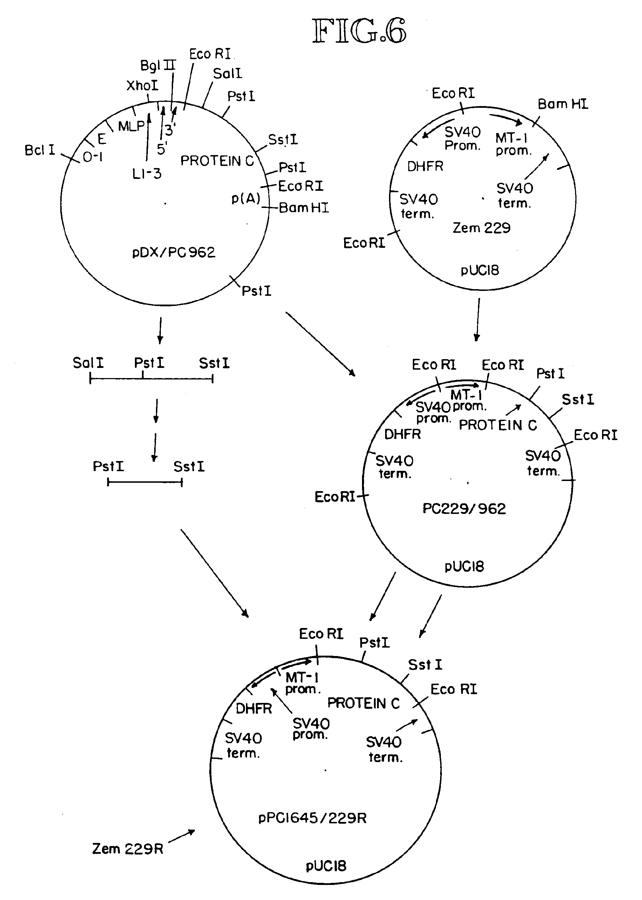

The mutant molecule was generated by altering the cloned cDNA by site-specific mutagenesis (essentially as described by Zoller and Smith, DNA 3:479-488, 1984) using the mutagenic oligonucleotide ZC962 (5' AGT CAC CTG AGA AGA AAA CGA GAC A 3') and oligonucleotide ZC550 (5' TCC CAG TCA CGA CGT 3'). Plasmid p594 was digested with Sst I, the approximately 87 bp fragment was cloned into M13mp11, and single-stranded template DNA was isolated. Following mutagenesis, a correct clone was identified by sequencing. Replicative form DNA was isolated and was digested with Sst I to isolate the mutagenized fragment. This mutagenized fragment was joined with Sst I-cut p594 in a two-part ligation. Clones having the Sst I fragment inserted in the desired orientation were identified by restriction enzyme mapping. The resulting expression vector was designated pDX/PC962 (Figure 6).

Plasmid pDX/PC962 was digested with Sal I and Sst I, and the purified 730 bp fragment was inserted into M13mp10 that had been linearized by digestion with Sal I and Sst I. Synthetic oligonucleotides ZC1645 (5' GAA GAC CAA ACA ACA AAA CGG CTC ATT GAT 3') and ZC550 were used to mutagenize the single-stranded template DNA by site-directed in vitro mutagenesis (Zoller and Smith, ibid.). The mutant phage clones were subjected to dideoxy-sequencing to confirm the mutagenesis. Replicative form (rf) DNA from a confirmed mutant phage clone, designated 1645, was prepared and was digested with Sst I and Pst I to isolate the 411 bp fragment.

Plasmid pDX/PC962 was digested with Eco RI, and the protein C fragment was recovered. This fragment was joined, via oligonucleotide adaptors, to phosphatase treated Bam HI-cut plasmid Zem229. The resulting plasmid, PC229/962 (Figure 6), was digested with Eco RI and Pst I to isolate the 592 bp protein C fragment. Plasmid PC229/962 was also digested with Eco RI and Sst I to isolate the 700 bp protein C fragment. The 411 bp protein C fragment from the 1645 rf, the 592 bp protein C fragment from PC229/962, and the 700 bp protein C fragment were joined in a four-part ligation with Zem229R that had been linearized with Eco RI and treated with calf intestinal phosphatase to prevent self-ligation. A correct plasmid was selected and was designated pPC1645/229R (Figure 6).

Plasmid pPC1645/229R was transfected into tk⁻ ts13 BHK cells by calcium phosphate co-precipitation (Graham and van der Eb, ibid.). Transfected cells were subjected to selection with 1 μM methotrexate and media were assayed for protein C by ELISA. A positive clone was grown in DMEM supplemented with 10% fetal calf serum and 1 μM methotrexate until the cells reached confluency. The confluent cells were switched to DMEM supplemented with 1% fetal calf serum and 1 μM methotrexate. Media were collected every 1 to 2 days over a period of 7 days and frozen at -20°C. The frozen media samples were thawed and filtered through 0.45 μm filters to remove any cell debris. Solid calcium chloride was added to a final concentration of 5 mM and solid sodium azide was added to a final concentration of 0.02% (weight/volume). Protein C was purified from the media using a monoclonal antibody column specific for the calcium-induced conformation of protein C. The treated media samples were applied to the column, and protein C was eluted with TBS containing 10 mM EDTA. Protein C concentrations were determined by absorbance at 280 nm and by ELISA.

Activated protein C produced from pPC1645/229R-transfected cells was compared to an equivalent amount of PC229/962 protein C using a chromogenic assay. One μg of affinity-purified protein C diluted in 40 μl TBS + EDTA was added to each well of a 96-well plate. Forty μl of 2 mM Spectrozyme PCa (American Diagnostica Inc, New York, NY) was added to each well and incubated at 37°C until there was sufficient color development. Activity was measured as an increase in absorbance at 405 nm. The results showed that the activated protein C produced from pPC1645/229R-transfected cells was 5-10% more active than the PC229/962 produced protein C.

## F. Construction and Expression of PPC1880/229R.

The 1645 DNA sequence was further modified to remove the first, second, seventh and eighth amino acids of the spacer peptide. Single-stranded 1645 template DNA was subjected to site-directed in vitro mutagenesis (Zoller and Smith, ibid.) using synthetic oligonucleotides ZC1880 (5'AAA CGA GAC ACA GAC CAA AGA AGA 3') and ZC550. Positive phage clones were subjected to dideoxy sequencing to confirm the mutagenesis. A positive clone was identified and was designated 1880 (Table 1).

Replicative form DNA prepared from clone 1880 was digested with Sst I and Pst I to isolate the approximately 0.4 kb fragment. Plasmid PC229/962 was digested with Eco RI and Pst I to isolate the 562 bp protein C fragment Plasmid PC229/962 was also digested with Eco RI and Pst I to isolate the 700 bp protein C fragment. The 411 bp protein C fragment from the 1880 rf, the 700 bp and 562 bp fragments from PC229/962 and Eco RI digested Zem229R were joined in a four-part ligation. A correct plasmid was selected and was designated pPC1880/229R.

Plasmid pPC1880/229R was transfected into tk⁻ ts13 BHK cells. Analysis of media samples from transfected cells showed that activated protein C was produced.

## G. Construction and Expression of pPC1954/229R.

The coding sequence for the spacer peptide in the 1645 sequence is altered to remove the second through seventh amino acid codons. Single-stranded 1645 template DNA is prepared and subjected to site directed in vitro mutagenesis using the synthetic oligonucleotides ZC1954 (5' GAG AAG AAA ACG AGA CCA AAG AAG AAA AC 3') and ZC550. Positive clones are sequenced to confirm the mutagenesis. A positive clone is selected and designated 1954 (Table 1).

Replicative form DNA is prepared from 1954 and digested with Sst I and Pst I to isolate the approximately 400 bp mutagenized protein C fragment. Plasmid PC229/962 is digested with Eco RI and Pst I and with Sst I and Eco RI to isolate the 562 bp Eco RI-Pst I fragment and the 700 bp protein C fragment. The approximately 0.4 kb protein C fragment from the 1954 rf, the 700 bp and 562 bp fragments from pPC229/962 and Eco RI-digested pZem229R are joined in a four-part ligation. A correct plasmid is selected and designated pPC1954/229R.

Plasmid pPC1954/229R is transfected into tk⁻ ts13 BHK cells by calcium phosphate co-precipitation (Graham and van der Eb, ibid.). Cells are selected and assayed for the production of activated protein C.

## H. Construction and Expression of pPC1953/229R.

The coding sequence for the spacer peptide in the 1645 sequence is altered to remove the first through eighth amino acid codons, resulting in a fusion between the first and second sets of Arg-Arg-Lys-Arg amino acid codons present in 1645. The amino acid sequence at the light-heavy chain junction of the encoded protein (designated 1953) is shown in Table 1.

Single-stranded 1645 template DNA is subjected to site directed in vitro mutagenesis using the synthetic oligonucleotides ZC1953 (5' ACC TCA GAA GAA AAC GAA GAA GAA AAC GGC TCA T 3') and ZC550. Positive clones are sequenced to confirm the mutagenesis. A positive clone is selected and is designated 1953. Replicative form DNA is prepared from clone 1953 and is digested with Sst I and Pst I to isolate the approximately 0.4 kb mutagenized protein C fragment. Plasmid PC229/962 is digested with Eco RI and Pst I or Sst I and Eco RI to isolate the 562 bp Eco RI-Pst I fragment and the 700 bp protein C fragment. The approximately 400 bp protein C fragment from the 1953 rf, the 700 bp and 562 bp fragments from PC229/962 and Eco RI digested Zem229R are joined in a four-part ligation. A correct plasmid is selected and designated pPC1953/229R

Plasmid pPC1953/229R is transfected into tk⁻ ts13 BHK cells by calcium phosphate co-precipitation (Graham and van der Eb, ibid.). Cells are selected and assayed for the production of activated protein C.

## I. Construction of pPC2043/ZMB-2

An activated protein C precursor is constructed in which the sequence encoding the activation peptide is removed and an Arg codon is inserted between amino acid codons 150 and 151 of native protein C. The amino acid sequence at the light-heavy chain junction of the encoded protein (designated 2043) is shown in Table 1.

Single stranded template DNA is prepared from phage clone 1962 and subjected to site-directed in vitro mutagenesis using the synthetic oligonucleotides ZC2043 (5' AGC CGG ATG GAG AAG AGG AAG CGC CTC ATT GC 3') and ZC550. Positive clones are sequenced to confirm the mutagenesis. Replicative form DNA is prepared from a confirmed phage clone and is digested with Pst I and Sst I to isolate the approximately 0.4 kb mutagenized fragment. Plasmid PC229/962 is digested with Eco RI and Pst I and with Sst I and Eco RI. The resulting 562 bp Eco RI-Pst I and 700 bp Eco RI-Sst I protein C fragments are recovered. Plasmid ZMB-2 is linearized by digestion with Eco RI. The 0.4 kb Pst I-Sst I fragment is joined with the 562 bp Eco RI-Pst I fragment, the 700 bp Sst I-Eco RI fragment and the linearized ZMB-2 in a four part ligation. A plasmid containing the insert in the correct orientation is designated pPC2043/ZMB-2.

Plasmid pPC2043/ZMB-2 is transfected into tk⁻ ts13 BHK cells. Transfected cells are assayed for the production of activated protein C.

## Claims

1. A method for producing activated protein C comprising:
   culturing mammalian cells stably transfected with an expression vector comprising a transcriptional promoter operably linked to a DNA sequence encoding activated protein C in a culture medium, and
   isolating the activated protein C produced by the cells, characterised in that said medium contains not more than 0.1% serum.

2. The method of claim 1 wherein said medium is essentially free of serum.

3. The method of claim 1 wherein said cells are baby hamster kidney cells.

4. The method of claim 1 wherein said DNA sequence further codes for the amino acid sequence $R_1$-$R_2$-$R_3$-$R_4$-X-$R_5$-$R_6$-$R_7$-$R_6$ between the light and heavy chains of said activated protein C, wherein each of $R_1$-$R_6$ is lysine or arginine and X is a peptide bond or spacer peptide of 1-12 amino acids.

5. The method of claim 1 wherein said DNA sequence further codes for the amino acid sequence $(R_1)_n$-$R_2$-$R_3$-$R_4$, wherein each of $R_1$, $R_2$, $R_3$ and $R_4$ is Lys or Arg and n = 0, 1, 2 or 3, between the light and heavy chains of said activated protein C.

6. The method of claim 5 wherein said DNA sequence codes for the amino acid sequence Arg-Arg-Lys-Arg between the light and heavy chains of said activated protein C.

7. The method of claim 1 wherein said cells are further transfected to express the Saccharomyces cerevisiae KEX2 gene.

8. The method of claim 1 wherein said culture medium further comprises vitamin K.

9. A method according to Claims 1, 2 and 6, wherein said cells are further transfected to express the Saccharomyces cerevisiae KEX2 gene.

10. The method of claim 9 wherein said cells are baby hamster kidney cells.

**Patentansprüche**

1. Verfahren für die Produktion von aktiviertem Protein C, welches umfaßt:
   Kultivieren von Säugetierzellen, die stabil transfiziert sind mit einem Expressionsvektor, der einen Transkriptionspromotor umfaßt, der operabel mit einer DNA-Sequenz verknüpft ist, die aktiviertes Protein C kodiert, in einem Kulturmedium und
   Isolieren des von den Zellen produzierten aktivierten Proteins C,
   dadurch gekennzeichnet, daß besagtes Medium nicht mehr als 0,1 % Serum enthält.

2. Verfahren nach Anspruch 1, wobei besagtes Medium im wesentlichen serumfrei ist.

3. Verfahren nach Anspruch 1, wobei besagte Zellen Babyhamster-Nierenzellen sind.

4. Verfahren nach Anspruch 1, wobei besagte DNA-Sequenz außerdem für die Aminosäuresequenz $R_1$-$R_2$-$R_3$-$R_4$-X-$R_5$-$R_6$-$R_7$-$R_8$ zwischen den leichten und schweren Ketten von besagtem aktivierten Protein C kodiert, wobei jedes von $R_1$-$R_8$ Lysin oder Arginin ist und X eine Peptidbindung oder ein spacer-Peptid mit 1-12 Aminosäuren ist.

5. Verfahren nach Anspruch 1, wobei besagte DNA-Sequenz außerdem für die Aminosäuresequenz $(R_1)_n$-$R_2$-$R_3$-$R_4$, wobei jedes von $R_1$, $R_2$, $R_3$ und $R_4$ Lys oder Arg ist und n = 0, 1, 2 oder 3 ist, zwischen den leichten und schweren Ketten von besagtem aktivierten Protein C kodiert.

6. Verfahren nach Anspruch 5, wobei besagte DNA-Sequenz für die Aminosäuresequenz Arg-Arg-Lys-Arg zwischen den leichten und schweren Ketten von besagtem aktivierten Protein C kodiert.

7. Verfahren nach Anspruch 1, wobei besagte Zellen außerdem transfiziert sind, um das Saccharomyces cerevisiae-KEX2-Gen zu exprimieren.

8. Verfahren nach Anspruch 1, wobei besagtes Kulturmedium außerdem Vitamin K umfaßt.

9. Verfahren nach den Ansprüchen 1, 2 und 6, wobei besagte Zellen außerdem transfiziert sind, um das Saccharomyces cerevisiae-KEX2-Gen zu exprimieren.

10. Verfahren nach Anspruch 9, wobei besagte Zellen Babyhamster-Nierenzellen sind.


**Revendications**

1. Procédé de production de protéine C activée, comprenant :
   la culture de cellules de mammifères transfectées de manière stable avec un vecteur d'expression comprenant un promoteur de transcription lié de manière fonctionnelle à une séquence d'ADN codant pour la protéine C activée dans un milieu de culture, et
   l'isolement de la protéine C activée produite par les cellules, caractérisé en ce que ledit milieu ne contient pas plus de 0,1 % de sérum.

2. Procédé suivant la revendication 1, dans lequel le milieu est pratiquement dépourvu de sérum.

3. Procédé suivant la revendication 1, dans lequel les cellules sont des cellules rénales de hamster nouveau-né.

4. Procédé suivant la revendication 1, dans lequel la séquence d'ADN code en outre pour la séquence d'amino-acides $R_1$-$R_2$-$R_3$-$R_4$-X-$R_5$-$R_6$-$R_7$-$R_8$ entre les chaînes légère et lourde de la protéine C activée, dans lequel chacun des groupes $R_1$-$R_5$ représente la lysine ou l'arginine et X représente une liaison peptidique ou un peptide intercalaire de 1 a 12 amino-acides.

5. Procédé suivant la revendication 1, dans lequel la séquence d'ADN code en outre pour la séquence d'amino-acides $(R_1)_n$-$R_2$-$R_3$-$R_4$, dans laquelle chacun des groupes $R_1$, $R_2$, $R_3$ et $R_4$ représente Lys ou Arg et n est égal à 0, 1, 2 ou 3, entre les chaînes légère et lourde de la protéine C activée.

6. Procédé suivant la revendication 5, dans lequel la séquence d'ADN code pour la séquence diamino-acides Arg-Arg-Lys-Arg entre les chaînes légère et lourde de la protéine C activée.

7. Procédé suivant la revendication 1, dans lequel les cellules sont transfectées en outre pour l'expression du gène <u>KEX2</u> de <u>Saccharomyces</u> <u>cerevisiae</u>.

8. Procédé suivant la revendication 1, dans lequel le milieu de culture comprend en outre de la vitamine K.

9. Procédé suivant les revendications 1, 2 et 6, dans lequel les cellules sont en outre transfectées pour l'expression du gène <u>KEX2</u> de <u>Saccharomyces</u> <u>cerevisiae</u>.

10. Procédé suivant la revendication 9, dans lequel les cellules sont des cellules rénales de hamster nouveau-né.

```
GGCTGTCATG GCGGCAGGAC GGCGAACTTG CAGTATCTCC ACGACCCGCC CCTGTGCCAG TGCCTCCA

-42     -40                                              -30
ATG TGG CAG CTC ACA AGC CTC CTG CTG TTC GTG GCC ACC TGG GGA ATT TCC GGC
MET Trp Gln Leu Thr Ser Leu Leu Leu Phe Val Ala Thr Trp Gly Ile Ser Gly

              -20                                        -10
ACA CCA GCT CCT CTT GAC TCA GTG TTC TCC AGC AGC GAG CGT GCC CAC CAG GTG
Thr Pro Ala Pro Leu Asp Ser Val Phe Ser Ser Ser Glu Arg Ala His Gln Val

                    -1  +1                                    10
CTG CGG ATC CGC AAA CGT GCC AAC TCC TTC CTG GAG GAG CTC CGT CAC AGC AGC
Leu Arg Ile Arg Lys Arg Ala Asn Ser Phe Leu Glu Glu Leu Arg His Ser Ser

                              20                                    30
CTG GAG CGG GAG TGC ATA GAG GAG ATC TGT GAC TTC GAG GAG GCC AAG GAA ATT
Leu Glu Arg Glu Cys Ile Glu Glu Ile Cys Asp Phe Glu Glu Ala Lys Glu Ile

                                   40
TTC CAA AAT GTG GAT GAC ACA CTG GCC TTC TGG TCC AAG CAC GTC GAC GGT GAC
Phe Gln Asn Val Asp Asp Thr Leu Ala Phe Trp Ser Lys His Val Asp Gly Asp

     50                                        60
CAG TGC TTG GTC TTG CCC TTG GAG CAC CCG TGC GCC AGC CTG TGC TGC GGG CAC
Gln Cys Leu Val Leu Pro Leu Glu His Pro Cys Ala Ser Leu Cys Cys Gly His

               70                                        80
GGC ACG TGC ATC GAC GGC ATC GGC AGC TTC AGC TGC GAC TGC CGC AGC GGC TGG
Gly Thr Cys Ile Asp Gly Ile Gly Ser Phe Ser Cys Asp Cys Arg Ser Gly Trp

                    90                         ♦        100
GAG GGC CGC TTC TGC CAG CGC GAG GTG AGC TTC CTC AAT TGC TCG CTG GAC AAC
Glu Gly Arg Phe Cys Gln Arg Glu Val Ser Phe Leu Asn Cys Ser Leu Asp Asn

                              110                                   120
GGC GGC TGC ACG CAT TAC TGC CTA GAG GAG GTG GGC TGG CGG CGC TGT AGC TGT
Gly Gly Cys Thr His Tyr Cys Leu Glu Glu Val Gly Trp Arg Arg Cys Ser Cys

                              130
GCG CCT GGC TAC AAG CTG GGG GAC GAC CTC CTG CAG TGT CAC CCC GCA GTG AAG
Ala Pro Gly Tyr Lys Leu Gly Asp Asp Leu Leu Gln Cys His Pro Ala Val Lys

     140                                        150
TTC CCT TGT GGG AGG CCC TGG AAG CGG ATG GAG AAG AAG CGC AGT CAC CTG AAA
Phe Pro Cys Gly Arg Pro Trp Lys Arg Met Glu Lys Lys Arg Ser His Leu Lys

              160                                        ↓170
CGA GAC ACA GAA GAC CAA GAA GAC CAA GTA GAT CCG CGG CTC ATT GAT GGG AAG
Arg Asp Thr Glu Asp Gln Glu Asp Gln Val Asp Pro Arg Leu Ile Asp Gly Lys
```

## FIG. 1

```
                                        180                              190
ATG ACC AGG CGG GGA GAC AGC CCC TGG CAG GTG GTC CTG CTG GAC TCA AAG AAG
Met Thr Arg Arg Gly Asp Ser Pro Trp Gln Val Val Leu Leu Asp Ser Lys Lys


                                   200                                   210
AAG CTG GCC TGC GGG GCA GTG CTC ATC CAC CCC TCC TGG GTG CTG ACA GCG GCC
Lys Leu Ala Cys Gly Ala Val Leu Ile His Pro Ser Trp Val Leu Thr Ala Ala


                                   220
CAC TGC ATG GAT GAG TCC AAG AAG CTC CTT GTC AGG CTT GGA GAG TAT GAC CTG
His Cys Met Asp Glu Ser Lys Lys Leu Leu Val Arg Leu Gly Glu Tyr Asp Leu


        230                                        240
CGG CGC TGG GAG AAG TGG GAG CTG GAC CTG GAC ATC AAG GAG GTC TTC GTC CAC
Arg Arg Trp Glu Lys Trp Glu Leu Asp Leu Asp Ile Lys Glu Val Phe Val His


        ♦     250                                  260
CCC AAC TAC AGC AAG AGC ACC ACC GAC AAT GAC ATC GCA CTG CTG CAC CTG GCC
Pro Asn Tyr Ser Lys Ser Thr Thr Asp Asn Asp Ile Ala Leu Leu His Leu Ala


                         270                              280
CAG CCC GCC ACC CTC TCG CAG ACC ATA GTG CCC ATC TGC CTC CCG GAC AGC GGC
Gln Pro Ala Thr Leu Ser Gln Thr Ile Val Pro Ile Cys Leu Pro Asp Ser Gly


                              290                                        300
CTT GCA GAG CGC GAG CTC AAT CAG GCC GGC CAG GAG ACC CTC GTG ACG GGC TGG
Leu Ala Glu Arg Glu Leu Asn Gln Ala Gly Gln Glu Thr Leu Val Thr Gly Trp


                                   310              ♦
GGC TAC CAC AGC AGC CGA GAG AAG GAG GCC AAG AGA AAC CGC ACC TTC GTC CTC
Gly Tyr His Ser Ser Arg Glu Lys Glu Ala Lys Arg Asn Arg Thr Phe Val Leu


        320                                   ♦ 330
AAC TTC ATC AAG ATT CCC GTG GTC CCG CAC AAT GAG TGC AGC GAG GTC ATG AGC
Asn Phe Ile Lys Ile Pro Val Val Pro His Asn Glu Cys Ser Glu Val Met Ser


             340                                   350
AAC ATG GTG TCT GAG AAC ATG CTG TGT GCG GGC ATC CTC GGG GAC CGG CAG GAT
Asn Met Val Ser Glu Asn Met Leu Cys Ala Gly Ile Leu Gly Asp Arg Gln Asp


             360                                   370
GCC TGC GAG GGC GAC AGT GGG GGG CCC ATG GTC GCC TCC TTC CAC GGC ACC TGG
Ala Cys Glu Gly Asp Ser Gly Gly Pro Met Val Ala Ser Phe His Gly Thr Trp


             380                                        390
TTC CTG GTG GGC CTG GTG AGC TGG GGT GAG GGC TGT GGG CTC CTT CAC AAC TAC
Phe Leu Val Gly Leu Val Ser Trp Gly Glu Gly Cys Gly Leu Leu His Asn Tyr


             400
GGC GTT TAC ACC AAA GTC AGC CGC TAC CTC GAC TGG ATC CAT GGG CAC ATC AGA
Gly Val Tyr Thr Lys Val Ser Arg Tyr Leu Asp Trp Ile His Gly His Ile Arg
```

FIG. 1 CONT.

410                                             419
GAC AAG GAA GCC CCC CAG AAG AGC TGG GCA CCT TAG CGACCCTCCC TGCAGGGCTG
Asp Lys Glu Ala Pro Gln Lys Ser Trp Ala Pro

GGCTTTTGCA TGGCAATGGA TGGGACATTA AAGGGACATG TAACAAGCAC ACCGGCCTGC TGTTCTGTCC

TTCCATCCCT CTTTTGGGCT CTTCTGGAGG GAAGTAACAT TTACTGAGCA CCTGTTGTAT GTCACATGCC

TTATGAATAG AATCTTAACT CCTAGAGCAA CTCTGTGGGG TGGGGAGGAG CAGATCCAAG TTTTGCGGGG

TCTAAAGCTG TGTGTGTTGA GGGGGATACT CTGTTTATGA AAAAGAATAA AAAACACAAC CACGAAAAAA

# FIG. 1 CONT.

# FIG.2

# FIG.3

FIG. 4

FIG.5

# FIG.6